# EUROPEAN PATENT SPECIFICATION

(11) **EP 4 054 537 B1**
(45) Date of publication and mention of the grant of the patent: **07.06.2023**
(21) Application number: 20801237.7
(22) Date of filing: 04.11.2020
(51) Int. Cl.: A61K 9/20, A61K 31/437, A61P 1/04

(54) **ORAL FORMULATION OF X842**
ORALE FORMULIERUNG VON X842
FORMULATION ORALE DE X842

(30) Priority: 04.11.2019 SE 1951255
(43) Date of publication of application: 14.09.2022
(73) Proprietor: Cinclus Pharma Holding AB (publ), 111 22 Stockholm (SE)
(72) Inventor: ANDERSSON, Kjell, 437 31 Lindome (SE); SPRINGFELTER, Mattias, 192 74 Sollentuna (SE); YOUSEF, Gabriella, 172 39 Sundbyberg (SE)
(74) Representative: Novitas Patent AB
(86) International application number: PCT/EP2020/080877
(87) International publication number: WO 2021/089580

(56) References cited:
- WO-A1-2010/063876
- UNGE PETER ET AL: "Sa1093 - A First-In-Human, Open-Label, Healthy Volunteer Study of the New P-Cab X842 Demonstrating 24H Acid Control for Treatment of Acid Related Diseases", GASTROENTEROLOGY, ELSEVIER INC, US, vol. 154, no. 6, 1 May 2018 (2018-05-01), XP085389846, ISSN: 0016-5085, DOI: 10.1016/S0016-5085(18)31174-0 cited in the application

## Description

The invention relates to an immediate release oral formulation of X842 comprising only limited amounts of surfactant. The invention also relates to the use of the oral formulation in the treatment of gastrointestinal inflammatory diseases or gastric acid related diseases, in particular erosive gastroesophageal reflux disease (eGERD).

### BACKGROUND

The compound 5-12-[(18-[(2,6-dimethylbenzyl)amino]-2,3-dimethylimidazo[1,2-a]pyridine-6-yl}carbonyl)-amino]ethoxy}-5-oxopentanoic acid (X842; structure shown below) is disclosed in WO 2010/063876. It is a potassium-competitive acid blocker (P-CAB), which competitively inhibits the gastric hydrogen potassium pump (H⁺/K⁺ ATPase) in the parietal cells. X842 may therefore be used to control the secretion of gastric acid in the stomach.

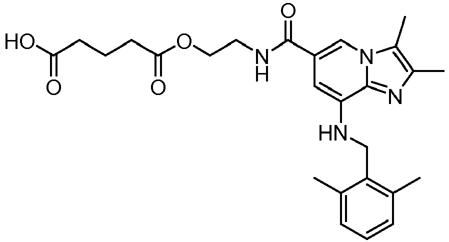

X842 is a prodrug of linaprazan, which was disclosed in WO 99/55706 and previously studied in Phase I and II studies. These studies showed that linaprazan was well tolerated, with a fast onset of action and full effect at first dose. However, linaprazan was quickly eliminated from the body and had too short duration of acid inhibition. In comparison, X842 has a longer half-life in the body and shows total control of the gastric acid production for a longer time compared to linaprazan. A clinical Phase I study has shown that administration of a single dose of X842 can maintain the intragastric acidity above pH 4 for 24 hours. X842 is therefore tailored for patients with severe erosive gastroesophageal reflux disease (eGERD).

The solubility of X842 is very low. It is practically insoluble in water at pH 6.8, whereas the solubility in water at pH 1.0 has been determined to be as low as about 0.113 mg/mL. This low solubility is problematic for solid oral formulations, and especially for solid immediate release formulations where the active ingredient should dissolve in only a short time period.

There is therefore a need for a formulation of X842 that rapidly dissolves in the stomach following oral administration. Such a formulation should be suitable for *H. pylori* eradication and for the treatment of gastric acid reflux disease, in particular for the treatment of severe erosive gastroesophageal reflux disease. It would be desirable if such a formulation would allow for a once daily administration.

### DETAILED DESCRIPTION OF THE INVENTION

It has been discovered that solid formulations of X842 are able to rapidly dissolve in aqueous solutions when the formulations comprise limited amounts of a pharmaceutically acceptable surfactant. In a first aspect, therefore, the invention relates to an oral formulation of X842 for immediate release, comprising:
a) a therapeutically effective amount of X842; and
b) a surfactant in an amount of about 12.0% (w/w) or less, relative to the amount of X842.

The surfactant may be a cationic surfactant, an anionic surfactant or a nonionic surfactant. Examples of cationic surfactants include, but are not limited to, cetyltrimethylammonium bromide (cetrimonium bromide) and cetylpyridinium chloride. Examples of anionic surfactants include, but are not limited to, sodium dodecyl sulfate (sodium lauryl sulfate) and ammonium dodecyl sulfate (ammonium lauryl sulfate). Examples of nonionic surfactants include, but are not limited to, glycerol monooleate, glycerol monostearate, polyoxyl castor oil (Cremophor EL), poloxamers (e.g., poloxamer 407 or 188), polysorbate 80 and sorbitan esters (Tween). In a preferred embodiment, the surfactant is an anionic surfactant. In a further preferred embodiment, the anionic surfactant is sodium dodecyl sulfate.

The surfactant may be present in the formulation in an amount of from about 1.0% (w/w) relative to the amount of X842, such as from about 2.0 to about 12.0% (w/w), such as from about 4.0 to about 12.0% (w/w), such as from about 6.0 to about 12.0% (w/w), such as from about 8.0 to about 12.0% (w/w), or such as from about 10.0 to about 12.0% (w/w) relative to the amount of X842. The amount of surfactant in the formulation is preferably aₛ low as possible. Therefore, in another embodiment, the formulation comprises from about 1.0 to about 11.0% (w/w), such as from about 1.0 to about 10.0% (w/w), such as from about 1.0 to about 9.0% (w/w), such as from about 1.0 to about 8.0% (w/w), or such as from about 1.0 to about 7.0% (w/w) of surfactant relative to the amount of X842. In another embodiment, the formulation comprises from about 2.0 to about 11.0% (w/w), such as from about 2.0 to about 10.0% (w/w), such as from about 2.0 to about 9.0% (w/w), such as from about 2.0 to about 8.0% (w/w), or such as from about 2.0 to about 7.0% (w/w) of surfactant relative to the amount of X842. In yet another embodiment, the formulation comprises from about 4.0 to about 11.0% (w/w), such as from about 4.0 to about 10.0% (w/w), such as from about 4.0 to about 9.0% (w/w), such as from about 4.0 to about 8.0% (w/w), or such as from about 4.0 to about 7.0% (w/w) of surfactant relative to the amount of X842.

In some embodiments, the formulation comprises surfactant in an amount of about 11.0% (w/w) or less, relative to the amount of X842. In some embodiments, the formulation comprises surfactant in an amount of about 10.0% (w/w) or less, relative to the amount of X842. In some embodiments, the formulation comprises surfactant in an amount of about 9.0% (w/w) or less, relative to the amount of X842. In some embodiments, the formulation comprises surfactant in an amount of about 8.0% (w/w) or less, surfactant relative to the amount of X842.

The oral formulation may additionally comprise one or more pharmaceutically acceptable excipients selected from the group consisting of fillers, disintegrants and lubricants.

Examples of suitable fillers include, but are not limited to, dicalcium phosphate dihydrate, calcium sulfate, lactose (such as lactose monohydrate), sucrose, mannitol, sorbitol, cellulose, microcrystalline cellulose, dry starch, hydrolyzed starches and pregelatinized starch. In certain embodiments, the filler is lactose, such as lactose monohydrate.

Examples of suitable disintegrants include, but are not limited to, dry starch, modified starch (such as (partially) pregelatinized starch, sodium starch glycolate and sodium carboxymethyl starch), alginic acid, cellulose derivatives (such as sodium carboxymethylcellulose, hydroxypropyl cellulose, and low substituted hydroxypropyl cellulose (L-HPC)) and cross-linked polymers (such as carmellose, croscarmellose sodium, carmellose calcium and cross-linked PVP (crospovidone)). In certain embodiments, the disintegrant is croscarmellose sodium.

Examples of suitable lubricants include, but are not limited to, talc, magnesium stearate, calcium stearate, sodium stearyl fumarate, stearic acid, glyceryl behenate, colloidal anhydrous silica, aqueous silicon dioxide, synthetic magnesium silicate, fine granulated silicon oxide, starch, sodium lauryl sulfate, boric acid, magnesium oxide, waxes (such as carnauba wax), hydrogenated oil, polyethylene glycol, sodium benzoate, polyethylene glycol, and mineral oil. In certain embodiments, the lubricant is sodium stearyl fumarate.

In another embodiment, the oral formulation comprises:
a) a therapeutically effective amount of X842;
b) an anionic surfactant in an amount of about 12.0% (w/w) or less, relative to the amount of X842; and
c) at least one filler;
d) at least one disintegrant; and
e) at least one lubricant.

In another embodiment, the oral formulation comprises:
a) a therapeutically effective amount of X842;
b) sodium dodecyl sulfate in an amount of about 12.0% (w/w) or less, relative to the amount of X842;
c) lactose monohydrate;
d) croscarmellose sodium; and
e) sodium stearyl fumarate.

In one embodiment, the oral formulation additionally comprises a glidant. Examples of suitable glidants include, but are not limited to, talc, starch, magnesium stearate, calcium stearate, colloidal anhydrous silica, synthetic magnesium silicate, fine granulated silicon oxide. In certain embodiments, the glidant is colloidal anhydrous silica.

In another embodiment, the formulation does not comprise microcrystalline cellulose.

The ingredients of the formulation are preferably mixed to a homogenous mixture and then formulated as tablets or capsules. The homogenous mixture of the ingredients may be compressed into tablets using conventional techniques, such as rotary tablet press. Alternatively, the mixture may be wetted by the addition of a liquid, such as water and/or an appropriate organic solvent (e.g., ethanol or isopropanol), and thereafter granulated and dried. The granules obtained may be then be compressed into tablets using conventional techniques. Capsules may comprise a powder mixture or small multiparticulates (such as granules, extruded pellets or minitablets) of the ingredients. In a preferred embodiment, the formulation is in the form of a tablet.

The amount of the X842 to be administered will be different for the patient being treated, and may vary from about 0.5 mg/kg to about 5 mg/kg of body weight per day, such as about 1.0 mg/kg per day, or about 1.5 mg/kg per day, or about 2.0 mg/kg per day, or about 2.5 mg/kg per day. The amount of X842 to be administered will depend on the severity of the disease, the age and weight of the patient and other factors normally considered by the attending physician, when determining the appropriate dosage level for the patient.

In one embodiment, a unit dose of the formulation comprises from about 25 to about 250 mg of X842, such as from about 50 to about 250 mg of X842, such as from about 50 to about 200 mg of X842, or such as from about 50 to 150 mg of X842. For example, a unit dose of the formulation may comprise about 30 mg, about 40 mg, about 50 mg, about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, or about 200 mg of X842. The daily dose can be administered as a single dose or divided into two, three or more unit doses.

The presence of a small amount of a surfactant (such as an anionic surfactant, e.g., sodium dodecyl sulfate) in the oral formulation allows the active ingredient (X842) to quickly dissolve in an aqueous environment, such as in the contents of the stomach. The rate of dissolution of X842 may be easily determined *in vitro* using a method based on European Pharmacopoeia 9.0, monograph 2.9.3, e.g. as described in the experimental section. Therefore, in one embodiment, the formulation disclosed herein displays at least 70% dissolution within 30 minutes, as measured using a Dissolution Apparatus 2 (paddle) Ph. Eur. 2.9.3. In a more preferred embodiment, the formulation displays at least 85% dissolution within 10 minutes, as measured using a Dissolution Apparatus 2 (paddle) Ph. Eur. 2.9.3. In another preferred embodiment, the formulation displays at least 95% dissolution within 15 minutes, as measured using a Dissolution Apparatus 2 (paddle) Ph. Eur. 2.9.3.

Following absorption into the blood stream, X842 is quickly metabolized into linaprazan, which is the active metabolite of X842. Whereas the plasma concentration of X842 is only very low and difficult to determine, the plasma concentration of linaprazan may be determined instead. Phase I studies have indicated that certain doses of X842 should be able to maintain the intra-gastric pH above 4 for 24 hours after administration. It is estimated that this requires a minimal plasma concentration (Cₘᵢₙ) of linaprazan of at least about 240 nmol/L after 22 hours. At such doses, a once daily administration of the formulation would be sufficient. Therefore, in another embodiment, the invention relates to a formulation as disclosed herein, wherein the formulation provides a Cₘᵢₙ of linaprazan in a human of at least about 240 nmol/L after 22 hours following administration of the oral formulation to said human. In a preferred embodiment, the invention relates to a formulation as disclosed herein, wherein a unit dose of the formulation provides a Cₘᵢₙ of linaprazan in a human of at least about 240 nmol/L after 22 hours following administration of the oral formulation to said human.

In a particular embodiment, the formulation has the following composition:

| **Ingredient** | | **Amount (mg)** | **Amount (%)** |
|---|---|---|---|
| *Active pharmaceutical ingredient* | | | |
| | X842 | 50.0 | 9.09 |

| *Excipients* | | | |
|---|---|---|---|
| | Lactose monohydrate | 465.6 | 84.7 |
| | Croscarmellose sodium | 22.0 | 4.00 |
| | Sodium dodecyl sulfate | 5.50 | 1.00 |
| | Sodium stearyl fumarate | 5.50 | 1.00 |
| | Colloidal anhydrous silica | 1.40 | 0.25 |
| *Total* | | *550* | *100* |

As used herein, the terms "effective amount" or "therapeutically effective amount" refer to a sufficient amount of X842 that, following administration to a subject, will relieve to some extent one or more of the symptoms of the disease or condition being treated. The result includes reduction and/or alleviation of the signs, symptoms, or causes of a disease, or any other desired alteration of a biological system. For example, an "effective amount" for therapeutic use is the amount of X842 required to provide a clinically significant decrease in disease symptoms. An appropriate "effective" amount in any individual case is determined using any suitable technique, such as a dose escalation study.

As used herein, the term "about" refers to a value or parameter herein that includes (and describes) embodiments that are directed to that value or parameter per se. For example, description referring to "about 20" includes description of "20". Numeric ranges are inclusive of the numbers defining the range. Generally speaking, the term "about" refers to the indicated value of the variable and to all values of the variable that are within the experimental error of the indicated value (e.g., within the 95% confidence interval for the mean) or within 10 percent of the indicated value, whichever is greater.

The oral formulation disclosed herein may be used in the treatment of prevention of diseases or conditions wherein inhibition of gastric acid secretion is necessary or desirable, such as in *H. pylori* eradication. Examples of such diseases and conditions include gastrointestinal inflammatory diseases and gastric acid related diseases, such as gastritis, gastroesophageal reflux disease (GERD), erosive gastroesophageal reflux disease (eGERD), *H. pylori* infection, Zollinger-Ellison syndrome, peptic ulcer disease (including gastric ulcers and duodenal ulcers), bleeding gastric ulcer, symptoms of gastroesophageal reflux disease (including heartburn, regurgitation and nausea), gastrinoma and acute upper gastrointestinal bleeding. In one aspect, therefore, the invention relates to the oral formulation disclosed herein for use in the treatment or prevention of a gastrointestinal inflammatory disease or a gastric acid related disease. In another aspect, the invention relates to the use of the oral formulation disclosed herein for the treatment or prevention of a gastrointestinal inflammatory disease or a gastric acid related disease. In yet another aspect, the invention relates to a method of treating or preventing a gastrointestinal inflammatory disease or a gastric acid related disease in a subject, such as man, comprising administering to the subject in need of such treatment or prevention a therapeutically effective amount of the oral formulation disclosed herein. In a particular embodiment, the treatment of GERD is on-demand treatment of GERD.

The invention is further illustrated by means of the following examples.

### EXAMPLES

### Example 1

### Preparation of the formulation

Tablets comprising 50 mg X842 were prepared on a 2 kg scale, using the amounts indicated in the table below.

| **Ingredient** | | **Amount (g/batch)** | **Amount (%)** |
|---|---|---|---|
| *Active pharmaceutical ingredient* | | | |
| | X842 | 182 | 9.09 |

| *Excipients* | | | |
|---|---|---|---|
| | Lactose monohydrate | 1693 | 84.7 |
| | Croscarmellose sodium | 80 | 4.00 |
| | Sodium dodecyl sulfate | 20 | 1.00 |
| | Sodium stearyl fumarate | 20 | 1.00 |
| | Colloidal anhydrous silica | 5 | 0.25 |
| *Total* | | *2000* | *100* |

Colloidal anhydrous silica and X842 were premixed and sieved through a stainless steel mesh into a mixing container. Lactose monohydrate, croscarmellose sodium and sodium dodecyl sulphate were sieved through a stainless steel mesh and added to the mixing container. Blending was performed in the mixing container for approximately 20 minutes.

Sodium stearyl fumarate was premixed with a small part of the powder blend, passed through a stainless steel mesh and added to the blending container. Blending was performed in the blending container for approximately 5 minutes.

The powder blend was then compressed into round, 550 mg tablets in a rotary tablet press. The compressed tablets were packed into plastic bottles and labelled.

### Example 2

### Dissolution test

Dissolution characteristics for the tablets of Example 1 were determined using Dissolution Apparatus 2 (paddle), as described in European Pharmacopeia 9.0, monograph 2.9.3. 1 tablet was added to a vessel containing 900 mLof a buffered solution of acetate (pH 4.5) containing 0.2 % SDS, and the contents were stirred at 37 ± 0.5 °C. Samples of the solution were withdrawn at different time points and the amount of dissolved X842 was quantified using an UV-spectrophotometer at 302 nm. Each experiment was repeated 6 times and the average values were calculated. The dissolution characteristics for tablets of Example 1 are shown in the table below.

| **Time (min)** | **Dissolution X842 (%)** | |
|---|---|---|
| | **Mean** | **Range** |
| 5 | 44 | 39-53 |
| 10 | 86 | 82-89 |
| 15 | 96 | 94-98 |
| 30 | 99 | 98-100 |

### Example 3

### Stability testing

Batches of the X842 tablets are stored in open HDPE bottles at 25°C and 60% relative humidity, or in closed HDPE bottles with an LDPE/HDPE cap, either at 25°C and 60% relative humidity (long-term storage conditions) or at 40°C and 75% relative humidity (accelerated conditions). The amounts of X842, degradation products and water, as well as the dissolution characteristics, will be determined at selected time intervals.

## Claims

1. An oral formulation of X842 for immediate release, comprising
a) a therapeutically effective amount of X842 (5-{2-[({8-[(2,6-dimethylbenzyl)amino]-2,3-dimethylimidazo[1,2-a]pyridine-6- yl}carbonyl)-amino]ethoxy}-5-oxopentanoic acid) and
b) a surfactant in an amount of from about 1.0 to about 12.0% (w/w) relative to the amount of X842.

2. The oral formulation according to claim 1, wherein the surfactant is an anionic surfactant.

3. The oral formulation according to claim 1 or 2, wherein the surfactant is sodium dodecyl sulfate.

4. The oral formulation according to any one of claims 1 to 3, wherein the formulation comprises about 11.0% (w/w) of surfactant relative to the amount of X842.

5. The oral formulation according to any one of claims 1 to 4, additionally comprising one or more excipients selected from the group consisting of fillers, disintegrants and lubricants.

6. The oral formulation according to any one of claims 1 to 5, comprising a filler which is lactose monohydrate.

7. The oral formulation according to any one of claims 1 to 6, comprising a disintegrant which is croscarmellose sodium.

8. The oral formulation according to any one of claims 1 to 7, comprising a lubricant which is sodium stearyl fumarate.

9. The oral formulation according to any one of claims 1 to 8, comprising
a) a therapeutically effective amount of X842;
b) sodium dodecyl sulfate in an amount of from about 1.0 to about 12.0% (w/w) relative to the amount of X842;
c) lactose monohydrate;
d) croscarmellose sodium; and
e) sodium stearyl fumarate.

10. The oral formulation according to any one of claims 1 to 9, wherein the formulation additionally comprises a glidant.

11. The oral formulation according to any one of claims 1 to 10, wherein the formulation does not comprise microcrystalline cellulose.

12. The oral formulation according to any one of claims 1 to 11, wherein the formulation displays at least 70% dissolution within 30 minutes, as measured using a Dissolution Apparatus 2 (paddle) Ph. Eur. 2.9.3.

13. The oral formulation according to any one of claims 1 to 12, wherein the formulation displays at least 85% dissolution within 10 minutes, as measured using a Dissolution Apparatus 2 (paddle) Ph. Eur. 2.9.3.

14. The oral formulation according to any one of claims 1 to 13, wherein the formulation displays at least 95% dissolution within 15 minutes, as measured using a Dissolution Apparatus 2 (paddle) Ph. Eur. 2.9.3.

15. The oral formulation according to any one of claims 1 to 14, wherein a unit dose of the formulation provides a Cₘᵢₙ of linaprazan in a human of at least about 240 nmol/L after 22 hours following administration of the oral formulation to said human.

16. The oral formulation according to any one of claims 1 to 15, for use in the treatment or prevention of a gastrointestinal inflammatory disease or a gastric acid related disease.

17. The oral formulation for use according to claim 16 in the treatment or prevention of gastritis, gastroesophageal reflux disease (GERD), erosive gastroesophageal reflux disease (eGERD), *H. pylori* infection, Zollinger-Ellison syndrome, peptic ulcer disease (including gastric ulcers and duodenal ulcers), bleeding gastric ulcer, symptoms of gastroesophageal reflux disease (including heartburn, regurgitation and nausea), gastrinoma and acute upper gastrointestinal bleeding.

## Patentansprüche

1. Orale Formulierung von X842 für eine sofortige Abgabe, umfassend
a) eine therapeutisch wirksame Menge von X842 (5-{2-[({8-[(2,6-Dimethylbenzyl)amino]-2,3-dimethylimidazo[1,2-a]pyridin-6-yl}carbonyl)-amino]ethoxy}-5-oxopentansäure) und
b) ein Tensid in einer Menge von etwa 1,0 bis etwa 12,0 % (Gew./Gew.) bezüglich der Menge von X842.

2. Orale Formulierung nach Anspruch 1, wobei das Tensid ein anionisches Tensid ist.

3. Orale Formulierung nach Anspruch 1 oder 2, wobei das Tensid Natriumdodecylsulfat ist.

4. Orale Formulierung nach einem der Ansprüche 1 bis 3, wobei die Formulierung etwa 11,0 % (Gew./Gew.) Tensid bezüglich der Menge von X842 umfasst.

5. Orale Formulierung nach einem der Ansprüche 1 bis 4, zusätzlich umfassend einen oder mehrere Trägerstoffe, die aus der Gruppe ausgewählt sind, bestehend aus Füllstoffen, Sprengmitteln und Schmiermitteln.

6. Orale Formulierung nach einem der Ansprüche 1 bis 5, umfassend einen Füllstoff, der Laktose-Monohydrat ist.

7. Orale Formulierung nach einem der Ansprüche 1 bis 6, umfassend ein Sprengmittel, das Croscarmellose-Natrium ist.

8. Orale Formulierung nach einem der Ansprüche 1 bis 7, umfassend ein Schmiermittel, das Natriumstearylfumarat ist.

9. Orale Formulierung nach einem der Ansprüche 1 bis 8, umfassend
a) eine therapeutisch wirksame Menge von X842;
b) Natriumdodecylsulfat in einer Menge von etwa 1,0 bis etwa 12,0 % (Gew./Gew.) bezüglich der Menge von X842;
c) Lactose-Monohydrat;
d) Croscarmellose-Natrium; und
e) Natriumstearylfumarat.

10. Orale Formulierung nach einem der Ansprüche 1 bis 9, wobei die Formulierung zusätzlich ein Gleitmittel umfasst.

11. Orale Formulierung nach einem der Ansprüche 1 bis 10, wobei die Formulierung keine mikrokristalline Cellulose umfasst.

12. Orale Formulierung nach einem der Ansprüche 1 bis 11, wobei die Formulierung mindestens 70 % Auflösung innerhalb von 30 Minuten zeigt, wie gemessen unter Verwendung einer Auflösungsvorrichtung 2 (Paddel) Ph. Eur. 2.9.3.

13. Orale Formulierung nach einem der Ansprüche 1 bis 12, wobei die Formulierung mindestens 85 % Auflösung innerhalb von 10 Minuten zeigt, wie gemessen unter Verwendung einer Auflösungsvorrichtung 2 (Paddel) Ph. Eur. 2.9.3.

14. Orale Formulierung nach einem der Ansprüche 1 bis 13, wobei die Formulierung mindestens 95 % Auflösung innerhalb von 15 Minuten zeigt, wie gemessen unter Verwendung einer Auflösungsvorrichtung 2 (Paddel) Ph. Eur. 2.9.3.

15. Orale Formulierung nach einem der Ansprüche 1 bis 14, wobei eine Einheitsdosis der Formulierung ein Cₘᵢₙ von Linaprazan in einem Menschen von mindestens etwa 240 nmol/L nach 22 Stunden im Anschluss an eine Verabreichung der oralen Formulierung an den Menschen bereitstellt.

16. Orale Formulierung nach einem der Ansprüche 1 bis 15 zur Verwendung bei der Behandlung oder Vorbeugung einer Magen-Darm-Entzündungskrankheit oder einer Magensäure-bezogenen Krankheit.

17. Orale Formulierung zur Verwendung nach Anspruch 16 bei der Behandlung oder Vorbeugung von Gastritis, gastroösophagealer Refluxkrankheit (GERD), erosiver gastroösophagealer Refluxkrankheit (eGERD), *H. pylori-*Infektion, Zollinger-Ellison-Syndrom, Ulcuskrankheit (einschließlich Magenulcera und Duodenalulcera), blutendem Magenulcus, Symptomen der gastroösophagealen Refluxkrankheit (einschließlich Sodbrennen, Regurgitation und Nausea), Gastrinom und akuter oberer Magen-Darm-Blutung.

## Revendications

1. Formulation orale de X842 pour libération immédiate, comprenant
a) une quantité thérapeutiquement efficace de X842 (acide 5-{2-[({8-[(2,6-diméthylbenzyl)amino]-2,3-diméthylimidazo[1,2-a]pyridin-6-yl}carbonyl)-amino]éthoxy}-5-oxopentanoïque) et
b) un agent tensioactif en une quantité allant d'environ 1,0 à environ 12,0 % (p/p) par rapport à la quantité de X842.

2. Formulation orale selon la revendication 1, dans laquelle l'agent tensioactif est un agent tensioactif anionique.

3. Formulation orale selon la revendication 1 ou 2, dans laquelle l'agent tensioactif est le dodécylsulfate de sodium.

4. Formulation orale selon l'une quelconque des revendications 1 à 3, dans laquelle la formulation comprend environ 11,0 % (p/p) d'agent tensioactif par rapport à la quantité de X842.

5. Formulation orale selon l'une quelconque des revendications 1 à 4, comprenant en outre un ou plusieurs excipients choisis dans le groupe constitué par des charges, des délitants et des lubrifiants.

6. Formulation orale selon l'une quelconque des revendications 1 à 5, comprenant une charge qui est le lactose monohydraté.

7. Formulation orale selon l'une quelconque des revendications 1 à 6, comprenant un délitant qui est la croscarmellose sodique.

8. Formulation orale selon l'une quelconque des revendications 1 à 7, comprenant un lubrifiant qui est le stéarylfumarate de sodium.

9. Formulation orale selon l'une quelconque des revendications 1 à 8, comprenant
a) une quantité thérapeutiquement efficace de X842 ;
b) du dodécylsulfate de sodium en une quantité allant d'environ 1,0 à environ 12,0 % (p/p) par rapport à la quantité de X842 ;
c) du monohydrate de lactose ;
d) de la croscarmellose sodique ; et
e) du stéarylfumarate de sodium.

10. Formulation orale selon l'une quelconque des revendications 1 à 9, dans laquelle la formulation comprend en outre un agent de glissement.

11. Formulation orale selon l'une quelconque des revendications 1 à 10, dans laquelle la formulation ne comprend pas de cellulose microcristalline.

12. Formulation orale selon l'une quelconque des revendications 1 à 11, dans laquelle la formulation présente au moins 70 % de dissolution en 30 minutes, telle que mesurée à l'aide d'un appareil de dissolution 2 (pale) Ph. Eur. 2.9.3.

13. Formulation orale selon l'une quelconque des revendications 1 à 12, dans laquelle la formulation présente au moins 85 % de dissolution en 10 minutes, telle que mesurée à l'aide d'un appareil de dissolution 2 (pale) Ph. Eur. 2.9.3.

14. Formulation orale selon l'une quelconque des revendications 1 à 13, dans laquelle la formulation présente au moins 95 % de dissolution en 15 minutes, telle que mesurée à l'aide d'un appareil de dissolution 2 (pale) Ph. Eur. 2.9.3.

15. Formulation orale selon l'une quelconque des revendications 1 à 14, dans laquelle une dose unitaire de la formulation fournit une Cₘᵢₙ de linaprazan chez un être humain d'au moins environ 240 nmol/L après 22 heures après l'administration de la formulation orale audit être humain.

16. Formulation orale selon l'une quelconque des revendications 1 à 15, pour une utilisation dans le traitement ou la prévention d'une maladie inflammatoire gastro-intestinale ou d'une maladie liée à l'acide gastrique.

17. Formulation orale pour une utilisation selon la revendication 16 dans le traitement ou la prévention de la gastrite, du reflux gastro-oesophagien pathologique (RGO pathologique), du reflux gastro-oesophagien pathologique érosif (RGO pathologique érosif), d'une infection par *H. pylori,* du syndrome de Zollinger-Ellison, de la maladie de l'ulcère gastroduodénal (y compris ulcères gastriques et ulcères duodénaux), de l'ulcère gastrique hémorragique, des symptômes du reflux gastro-oesophagien pathologique (y compris brûlures d'estomac, régurgitation et nausée), d'un gastrinome et de saignements gastro-intestinaux hauts aigus.
